# EUROPEAN PATENT SPECIFICATION

(11) **EP 0 388 171 B1**
(45) Date of publication and mention of the grant of the patent: **31.05.1995**
(21) Application number: 90302704.3
(22) Date of filing: 14.03.1990
(51) Int. Cl.: C12Q 1/68

(54) **Methods for purification, amplification and detection of a nucleic acid**
Verfahren zur Reinigung, Vervielfachung und zum Nachweis einer Nukleinsäure
Procédé de purification, amplification et détection d'un acide nucléique

(30) Priority: 17.03.1989 US 325311; 05.02.1990 US 475068
(43) Date of publication of application: 19.09.1990
(73) Proprietor: Johnson & Johnson Clinical Diagnostics, Inc., Rochester New York 14650 (US); CETUS ONCOLOGY CORPORATION, Emeryville California 94608 (US)
(72) Inventor: Oakes, Fred Terry, c/o Eastman Kodak Co., Rochester, NY 14650 (US); Burdick, Brent Arthur, c/o Eastman Kodak Co., Rochester, NY 14650 (US); Fyles, Janet Linn, c/o Eastman Kodak Co., Rochester, NY 14650 (US); Chang, Chu-an, c/o Eastman Kodak Co., Rochester, NY 14650 (US)
(74) Representative: Mercer, Christopher Paul

(56) References cited:
- EP-A- 0 184 056
- EP-A- 0 200 113
- EP-A- 0 258 017
- EP-A- 0 265 244
- EP-A- 0 296 557
- EP-A- 0 301 899
- EP-A- 0 317 074
- GB-A- 2 202 328
- NATURE, vol. 324, 13 November 1986; R.K. SAIKI et al., pp. 163-166#

## Description

The present invention relates to a method for purification of a predetermined nucleic acid in a biological specimen. It also relates to a method of amplifying the purified nucleic acid as well as its detection in analytical procedures.

Progress in biochemistry, molecular biology, genetic engineering and various diagnostic procedures often requires rapid and simple techniques for purifying and separating nucleic acids. For example, in genetic engineering, it is often desirable to be able to obtain a pure nucleic acid segment from a complex mixture of many different nucleic acids. In diagnosing the presence of certain infectious diseases, target DNA or fragments thereof may be present in such small concentrations that purification is desired prior to detection. A single nucleic acid is generally characterized by nucleotide sequence, molecular weight, size and shape.

Nucleic acid probe technology has developed rapidly in recent years as researchers have discovered its value for detection of various diseases, organisms or genetic features which are present in very small quantities in a test sample. The use of probes is based upon the concept of complementarity. For example, DNA is double-stranded, the strands bound to each other by hydrogen bonds between complementary nucleotides (also known as nucleotide pairs).

The DNA complex is normally stable, but the strands can be separated (or denatured) by conditions which disrupt the hydrogen bonding. The released single strands will reassociate only with another strand having a complementary sequence of nucleotides. This hybridization process can occur in solution or on a solid substrate. RNA is usually single-stranded. It will also hybridize with another strand or portion thereof which has a complementary sequence of nucleotides.

A target nucleic acid sequence of the DNA or RNA of a target organism or cell may be only a small portion of the total strand, so that it is very difficult to detect its presence using most known labeled probes. Much research has been carried out to overcome this problem including improvements in probe sensitivity and synthesis of nucleic acids.

A significant advance in the art is the process described in US-A-4,683,202. Without going into extensive detail regarding that process, it is an amplification technique wherein primers are hybridized to nucleic acid templates in the presence of a polymerization agent (such as a polymerase) and four nucleotide triphosphates, and extension products are formed from the primers. These products are denatured and used as templates in a cycling reaction which amplifies the number and amount of existing nucleic acids to facilitate their subsequent detection. The amplification process can be carried out cyclically as many times as desired to produce a larger quantity of detectable material from a small amount of target nucleic acid sequence.

While the amplification process described above is highly sensitive, in many instances, the amount of DNA material of interest in a biological specimen is very small. Considerable background from nonspecific nucleic acids or cellular debris may decrease assay sensitivity. Yet, it may not be possible to remove such materials without considerable effort and time, in which case the assay is delayed and any desired diagnosis and treatment is also delayed.

To eliminate undesired cellular debris and nucleic acids and thus enhance amplification and detection of predetermined nucleic acids, one may use any of a number of known methods, including those described in Blin et al, Nucleic Acid Res., 3, pp. 2302-2308 (1976), Marmur, J. Mol. Biol., 3, pp. 208-218 (1961), Birnboin et al, Nucleic Acid Res., 7, pp. 1513-1523 (1979), Bowtell, Anal. Biochem, 162, pp. 463-456 (1987), Beji et al, Anal. Biochem, 162, pp. 18-32 (1987), Buffone et al, Clin. Chem., 31, pp. 164-165 (1985), and others too numerous to mention.

Purification of nucleic acids using oligonucleotides which are covalently attached to polymeric particles are described, for example, in EP-A-0 296 557, EP-A-0 200 113, EP-A-0 265 244 and GB-A-2,202,328.

US-A-4,672,040 describes the use of magnetic particles for separation of molecules, macromolecules or nucleic acids from other materials in specimens. While this method may be useful for sophisticated clinical environments, it would be preferred to have a rapid and simple purification method which will enable nucleic acid separation in doctor's offices, clinics and other situations, prior to amplification, where a diagnostic result is desired quickly and efficiently. It is with such problems and features in mind that the present invention is advantageous.

The problems noted above are overcome with a method for amplifying at least one single-stranded, targeted nucleic acid in a biological specimen suspected of containing a mixture of nucleic acids using a polymerase chain reaction, the method comprising:
A. contacting the specimen with a purification reagent comprising a nonporous, nonmagnetic particle having directly attached thereto a nucleic acid fragment complementary to a nucleic acid sequence of the targeted nucleic acid, under conditions suitable to form an insoluble hybrid,
B. separating the hybrid from the remainder of the specimen, and
C. amplifying the nucleic acid sequence of interest using a polymerase chain reaction with or without denaturation from the insoluble hybrid.

This invention also provides a method for the detection of at least one targeted nucleic acid having two complementary strands present in a biological specimen suspected of containing a mixture of nucleic acids, the method comprising:
A. denaturing the complementary strands in the specimen,
B. contacting the specimen with a purification reagent comprising a nonporous, nonmagnetic particle having directly attached thereto a nucleic acid fragment complementary to a nucleic acid sequence of the targeted nucleic acid, under conditions suitable to form an insoluble hybrid,
C. separating the hybrid from the remainder of the specimen to provide purified targeted nucleic acid,
D. denaturing the hybrid and separating the insoluble complementary fragment from the targeted nucleic acid,
E. contacting a sample of the denatured targeted nucleic acid with first and second primers, one of which is complementary to the nucleic acid sequence of interest and the other which is complementary to the other strand of the targeted nucleic acid so as to form hybridized products of the first and second primers and the complementary nucleic acid strands,
F. forming first and second extension products of the primers in the hybridized product, which extension products, when separated from their complements, can serve as templates for synthesis of the extension products of the primers,
G. separating the primer extension products from the templates on which they were synthesized,
H. contacting the separated extension products and the targeted nucleic acid with additional first and second primers, resulting in amplification of the specific nucleic acid sequence to form complementary products,
I. separating the primer extension products from the complementary products formed in step H,
J. contacting at least one primer extension product separated in step I with an oligonucleotide probe which is labeled for detection and is complementary thereto to form a complementary product of the probe and the primer extension product, and
K. detecting the complementary product formed in step I as an indication of the presence of the targeted nucleic acid in the specimen.

The present invention provides an advantageous means for purifying a desired nucleic acid mixture of acids without the use of porous or magnetic particles as taught in the art, or other sophisticated equipment or reagents. While affinity separations are known for nucleic acids, it has not heretofore been used in order to improve the amplification of very small amounts of target nucleic acids using polymerase chain reaction. Thus, an already highly useful procedure is made even more desirable and sensitive with the present invention. These desirable results are achieved using a purification reagent composed of a complementary nucleic acid fragment directly attached to a nonporous, nonmagnetizable particle of suitable shape and size.

The present invention is directed to the amplification and detection of one or more targeted nucleic acids (that is, predetermined) in a test specimen. Such samples can include cellular or viral material, hair, body fluids or other materials containing genetic DNA or RNA which can be detected.

Nucleic acids can be obtained from various sources including plasmids, naturally occurring DNA or RNA from any source (such as bacteria, yeast, viruses, plants and higher animals, humans). They may be extracted from various tissues including blood, tissue material or other sources known in the art using known procedures. The present invention is particularly useful for the detection of nucleic acid sequences found in viruses or cells of any organism, such as in genomic DNA, bacterial DNA, viral RNA, or DNA or RNA found in bacterial or viral infected cells. This invention is particularly useful for the detection of proviral DNA from cells infected by HIV-I or other retroviruses.

According to the present invention, the purified nucleic acid is used in a chain reaction for producing, in exponential quantities relative to the number of reaction steps involved, at least one specific nucleic acid. The product will be a discrete nucleic acid duplex with termini corresponding to the ends of the specific primers employed. Any source of nucleic acid can be utilized as the starting material provided it contains or is suspected of containing the specific nucleic acid targeted for detection. By "nucleic acid" to be purified and duplicated is meant a fragment or the entire nucleic acid. Moreover, more than one nucleic acid can be purified and amplified simultaneously by using a specific set of purification reagents, primers and labeled probes (described below) for each targeted nucleic acid.

As used herein in referring to primers, probes or nucleic acid fragments to be detected, the term "oligonucleotide" refers to a molecule comprised of two or more deoxyribonucleotides or ribonucleotides, and preferably more than three. The exact size is not critical but depends upon many factors including the ultimate use or function of the oligonucleotide. The oligonucleotide may be derived synthetically or by cloning.

The term "primer" refers to an oligonucleotide, whether naturally occurring or synthetically produced, which is capable of acting as a point of initiation of synthesis when placed under conditions in which synthesis of a primer extension product complementary to a nucleic acid strand is induced. Such conditions include the presence of nucleotides (such as the four standard deoxyribonucleoside triphosphates) and an agent for polymerization such as a DNA polymerase, and suitable temperature and pH.

In the practice of this invention, primers, probes and fragments are substantially complementary to a specific nucleic acid sequence of the targeted nucleic acid. By "substantially complementary" is meant that there are a sufficient number of bases on complementary materials that match so that hybridization will occur. It does not mean, however, that every base pair will match.

The purification reagents useful in this invention comprise a nucleic acid fragment which is substantially complementary to at least one nucleic acid sequence of the targeted nucleic acid. This fragment can be of any suitable length that will provide suitable complementarity and hybridization with the target. Generally, the length of the fragment is at least 10 bases, and preferably from 15 to 50 bases. The fragments are tailored for at least one sequence of the targeted nucleic acid. It is also possible to have a mixture of purification reagents, each reagent having a particular fragment, but each fragment in the mixture being capable of hybridizing with the same or different sequence of the targeted nucleic acid. Useful fragments can be obtained from a number of sources or prepared using known techniques and equipment such as those described below for primer preparation.

The nucleic acid fragments just described are directly attached in a suitable manner to polymeric particles (described below). Attachment may be by chemical or physical means (that is, adsorption or covalent reaction), and it is directly from nucleotide to support. That is, there is no intermediate linking material between the particle and the fragment. In one embodiment, the nucleic acids are adsorbed to the surface of the polymeric particles. However, it is preferred to chemically modify the nucleic acid fragment to provide reactive groups which covalently bind to corresponding reactive groups on the solid support. Many such methods are known in the art, and include those described in WO-A-89/02931. More details about this method are provided in Example 1 below.

Useful particles for preparing the purification reagent can be made of any suitable material, including cellulosic materials, glass, ceramics, naturally occurring or synthetic polymers, metals and others readily apparent to one skilled in the art. However, such particles are not magnetic or magnetizable because they may inhibit polymerase activity. They can be of any useful shape and size including spherical, ellipsoidal, cubic, irregular or flat shape and having an average size of from 0.3 to 3 »m.

For covalent attachment, preferred polymeric particles used in preparing the purification reagent advantageously have reactive surface groups to which the fragment (modified or unmodified) can be reacted. Useful reactive groups include carboxy, amino, sulfhydryl, aldehyde, activated 2-substituted ethylsulfonyl, vinylsulfonyl, active halogen atoms, nitroaryl, esters and others readily apparent to one skilled in the art. Particularly useful groups include carboxy, esters, active halogen atoms, vinylsulfonyl and activated 2-substituted ethylsulfonyl as part of ethylenically unsaturated polymerizable monomers described in detail in EP-A-0 302 715.

The purification reagent described herein is used to hybridize with the targeted nucleic acid and thereby render the nucleic acid insoluble and separable from the remainder of the specimen. This contact of specimen and reagent is done under hybridization conditions which are well known in the art, and generally require at least 1 minute, moderate agitation, a pH range of from 5 to 9 and a temperature from 0 to 75°C. A skilled worker in the art would be able to adjust the conditions for optimal hybridization. A representative procedure is described in Example 1 below.

After hybridization, it is usually desirable to separate the insolubilized targeted nucleic acid from the specimen using an appropriate separation technique, such as centrifugation, filtration, selective adsorption to an insoluble matrix, sedimentation or washing of bound species. Centrifugation or filtration is preferred.

In the amplification and detection methods of this invention, useful primers can be obtained from a number of sources or prepared using known techniques and equipment, including for example, an ABI DNA Synthesizer (available from Applied Biosystems) or a Biosearch 8600 Series or 8800 Series Synthesizer (available from Milligen-Biosearch, Inc.) and known methods for their use. Naturally occurring primers isolated from biological sources are also useful (such as restriction endonuclease digests).

In some embodiments, at least one of the primers (or sets thereof) used in the detection method is labeled with a specific binding ligand. The term "labeled" refers to the fact that the ligand is attached to this primer in a manner such that it will not readily be detached. The specific binding ligand can be biotin or a derivative thereof, avidin, streptavidin or a derivative thereof, a lectin, a sugar, a protein, a hapten, a drug, or an immunological species, such as an antibody or an antigenic material.

The present invention is useful for amplification or detection of a targeted purified nucleic acid having two complementary strands. Most nucleic acid sequences of interest already are double-stranded, such as those found in DNA. However, single-stranded nucleic acid sequences, such as mRNA, can be similarly amplified and detected.

A specific nucleic acid sequence is produced using the nucleic acid containing that sequence as a template. If the nucleic acid contains two strands, it is necessary to separate the strands (called denaturation), either as a separate step or simultaneously with the formation of primer extension products. Denaturing can be accomplished using any suitable physical, chemical or enzymatic means as described in the art. Heating to a suitable temperature is a preferred means.

Once the separated strands are available for use, synthesis of additional nucleic acid strands can be carried out using two or more primers (at least one of which is labeled as described above) in a buffered aqueous solution at a pH of from 7 to 9. Preferably, a molar excess of the two primers is added to the buffered solution, and specific amounts are taught in the art. The deoxyribonucleoside triphosphates dATP, dCTP, dGTP and dTTP are also added to the synthesis mixture in adequate amounts and the resulting solution is heated to 90-100°C for up to 10 minutes, and preferably from 1 to 4 minutes. Enzyme cofactors, such as magnesium or manganese ions, are also preferably present in molar excess to the triphosphates. After this heating, the solution is preferably cooled to room temperature, and an appropriate agent for inducing (or catalyzing) the formation of primer extension products is introduced. This inducing agent is generally known in the art as a polymerization agent. Reaction to form these products is carried out under known conditions (generally from room temperature to that temperature at which polymerization no longer occurs).

The polymerization agent may be any compound, or combination of reagents, which will function to accomplish the synthesis of primer extension products, including enzymes (for example, E. coli DNA polymerase I, T4 DNA polymerase, Klenow polymerase, reverse transcriptase and others known in the art). Particularly useful enzymes are thermally stable enzymes, cloned or naturally occurring, such as those obtained from various Thermus bacterial species. Other polymerization agents are described in US-A-4,683,202 (noted above).

Preferred thermal-stable enzymes are DNA polymerases from Thermus aquaticus such as described in EP-A-0 258 017. Other useful enzymes are described by Rossi et al, Syst. Appl. Microbiol. 7(2-3), pp. 337-341, 1986. Many useful polymerases are commercially available. Generally, the synthesis of extension products will be initiated at the 3′ end of each primer and proceed in the 5′ to 3′ direction along the template until synthesis is terminated. Some polymerization agents (for example, reverse transcriptase) may proceed in the 3′ to 5′ direction along the template.

The newly formed primer extension products comprising the newly synthesized strands and their respective primers form double-stranded molecules with the initial target strands which are used in the succeeding steps of the method. These strands are then separated by denaturation as described above to provide single-stranded molecules, onto which new nucleic acids are synthesized as described above. Additional reagents may be needed to keep the amplification procedure going, after which most of the extension products will consist of the specific nucleic acid sequence bounded by the two primers (that is, complementary products).

The steps of strand separation and extension product synthesis can be repeated as often as needed to produce the desired quantity of the specific nucleic acid needed for the use, for example detection. Generally, the sequence of steps is repeated at least once, and preferably at least 10 to 50 times.

When it is desired to produce more than one targeted purified nucleic acid, the appropriate number of sets of primers are used in the general procedure described above.

At any point in the method of this invention after the generation of at least one primer extension product, that product can be hybridized with a detectably labeled probe (described below).

Various detection procedures can be used to determine the presence of the detectable hybrid including Southern blot, gel electrophoresis, staining and others known in the art.

Generally, once a desired amount of the nucleic acid sequence of interest has been generated and the primer extension products are separated for a last time, the first primer extension product is contacted with an oligonucleotide probe which is labeled for detection and is complementary thereto to form a product. The probe is a nucleic acid sequence which is complementary with the targeted nucleic acid sequence. The probes can be of any suitable length of nucleic acids, but preferably it is from 15 to 40 nucleotides. It is labeled (commonly at the 5′ end) with any suitable detectable material which will not interfere with the complexation of the specific binding ligand and its receptor. Procedures for attaching labels and preparing probes is well known in the art, for example, as described by Agrawal et al, Nucleic Acid Res., 14, pp. 6227-45 (1986), and in the references noted above for attaching a specific binding ligand to a primer. Useful labels include radioisotopes, electron-dense reagents, chromogens, fluorogens, phosphorescent moieties, ferritin and other magnetic particles, chemiluminescent moieties and enzymes (which are preferred). Useful enzymes include, glucose oxidase, peroxidase, uricase, alkaline phosphatase and others known in the art. Substrates and dye forming compositions for such enzymes are well known.

In a particularly preferred embodiment, the label is peroxidase, and at some point in the assay, hydrogen peroxide and suitable dye-forming compositions are added to provide a detectable dye. For example, useful dye-providing reagents include tetramethylbenzidine and derivatives thereof, and leuco dyes, such as triarylimidazole leuco dyes (as described in US-A-4,089,747, or other compounds which react to provide a dye in the presence of peroxidase and hydrogen peroxide.

Detection of the presence of the probe which is in the complementary product can be achieved using suitable and known detection equipment and procedures. Certain probes may be visible to the eye without the use of detection equipment. It is also useful for the method to be carried out in a suitable container. The most crude container would be a test tube, flask or beaker, but more sophisticated containers have been fashioned in order to facilitate automated procedures for performing the method. For example, a cuvette constructed to provide certain temperature characteristics during the practice of the method is described in EP-A-0 318 255. Other useful containers could be suitably fashioned for automated or single use of the method of this invention.

In order for the probe in the complementary product to be detected, it is often important for the complementary product to be separated from the other materials in the reaction medium. This can be done by suitable insolubilization means, such as by using a primer or probe which is attached or capable of becoming attached to a solid material at some point in the method. The resulting insolubilized complexed product can be separated from uncomplexed materials by filtration, centrifugation or other suitable separation techniques.

Particularly useful separation means are microporous filter membranes such as the polyamide membranes marketed by Pall Corp. (for example as LoProdyne™ or Biodyne™ membranes). They can be used uncoated or precoated with surfactants or other materials which facilitate the analytical procedures. In one embodiment, the membrane itself can have the receptor molecules attached thereto (by absorption or covalent bonds) for capturing the first primer extension products.

The membranes can be used as a separate substrate with suitable containers for carrying out other steps of the assay. Preferably, however, it is mounted as part of a test device. Various test devices are known in the art including those described in US-A-3,825,410, US-A-3,888,629, US-A-3,970,429 and US-A-4,446,232. Particularly useful devices are described in EP-A-0 308 231.

The method described herein can be used to provide the detection or characterization of specific nucleic acid sequences associated with infectious diseases, genetic disorders or cellular disorders such as cancers. It may also be used in forensic investigations and DNA typing. For purposes of this invention, genetic diseases include specific deletions or mutations in genomic DNA from any organism, such as sickle cell anemia, cystic fibrosis, α-thalassemia, β-thalassemia and others readily apparent to one skilled in the art. Various infectious diseases can be diagnosed by the presence in a clinical sample of small quantities of specific DNA sequences characteristic of the organism, whether it be a yeast, bacterium or virus. Such bacteria which can be detected include, but are not limited to, Salmonella, Chlamydia, Gonorrhea, Shigella and Listeria. Viruses which are detectable include, but are not limited to, herpes, Cytomegalovirus, Epstein-Barr virus, hepatitis and retroviruses such as HTLV-I and HIV-I. Protozoan parasites, yeasts and molds are also detectable. Other detectable species would be readily apparent to one skilled in the art. The invention is particularly useful for the detection of the presence of retroviruses, such as HIV-I, in test samples.

The following examples are included to illustrate the practice of this invention.

### Preparation and Use of a Purification Reagent

A purification reagent useful in the practice of this invention was prepared using polymeric latex particles prepared using standard techniques, and a nucleic acid fragment complementary to Human Leucocyte Antigen (HLA) DNA, types 2 and 3 regions, having the following sequence:
5'-X-CCTCTGTTCCACAGACTTAGATTTG-3'
wherein X is a free amino group attached to the oligonucleotide through an ethylene glycol spacer arm prepared as described in WO-A-89/02931.

### Materials:

The latex particles (2.9% solids) were comprised of poly(styrene-co-acrylic acid) (90:10 molar ratio).

Methylimidazole buffer (o.2 molar, pH 6), adjusted to pH 6 by adding prechloric acid.

SSPE buffer solution (2%) comprised 43.5 g sodium chloride, 0.276 g sodium phosphate and 0.074 g ethylene diaminetetraacetic acid in 1 liter water, and the pH adjusted to 7.4.

A solution of 5'-aminooligonucleotide (200 mmolar) in glass distilled water (40 OD/ml) was used.

EDC reagent is 1-(3-dimethylaminopropyl)-3-ethylcarbodiimide hydrochloride.

### Procedure:

The latex solution (1 ml, 29 mg beads) was centrifuged (2.5 minutes at 13,000x) to remove the supernatant. The beads were then resuspended in glass-distilled water (1 ml) by vigorous vortexing. The mixture was again centrifuged and the supernatant removed. The beads were washed once with methyl-imidazole buffer (1 ml), the buffer was removed by centrifugation, and the beads were resuspended in the same buffer (0.5 ml).

To the bead solution was added the 5'-aminooligonucleotide probe (25 »l of the stock solution, or about 5000 pmoles), and the resulting solution was mixed well. The 1-(3-dimethylaminopropyl)-3-ethylcarbodiimide hydrochloride reagent (15 mg) was added to the solution. After thorough mixing by vortex, the reaction mixture was left at 20-25°C with occasional mixing for at least 2 hours (usually for 15 hours).

The supernatant was first removed by centrifugation and the beads were subsequently washed with the following buffer solutions by centrifugation and pipetting off the supernatant between solutions:
a. once with 1 ml glass-distilled water,
b. once with the SSPE buffer solution,
c. once with phosphate buffered saline solution, and
d. twice with glass-distilled water.

After the last treatment, the beads were resuspended in glass-distilled water to a final volume of 92 »l to make a 3% bead suspension and kept at 4°C.

### Example 1: Method to Amplify and Detect HLA DNA

This example illustrates the amplification and detection of a purified targeted HLA DNA.

Crude whole blood lysate was prepared from a crude whole blood sample (100 »l) by boiling, centrifugation and withdrawing the supernatant. Portions (5 »l of each) of the crude lysate were heat denatured at 95°C for 5 minutes each, and then added to a sample (40 »l) of SSPE buffer solution and Triton™ X-100 nonionic surfactant (0.02 weight %) to provide a test specimen.

### Purification Procedure:

The purification reagent described above (2 »l at 2 weight % in water) was added to the test specimen described above, and the resulting mixture was incubated at 55°C for 30 minutes with agitation. The mixture was then centrifuged for one minutes at 14,000 rpm, and the supernatant discarded. The resulting pallet was resuspended in a solution (50 »l) of SSPE buffer solution and Triton™ X-100 nonionic surfactant (0.02 weight %), heated to 55°C and centrifuged again. This procedure was repeated twice more to provide a purified nucleic acid which is hybridized to the purification reagent.

The resulting pellet was then suspended in the following solution (100 »l): potassium chloride (50 mmolar), tris(hydroxymethyl)aminomethane buffer (10 mmolar, pH 8), gelatin (0.1 mg/ml), magnesium chloride (2.5 mmolar), HLA primers (- and +, 0.2 »molar each) and dNTP's (0.67 mmolar each).

The HLA DNA primers had the following sequences:
(+) 5'-X-GTGCTGCAGGTGTAAACTTGTACCAG-3'
(-) 5''-X-CGGATCCGGTAGCAGCGGTAGAGTTG-3'
wherein X represents a biotintetraethylene glycol linker prepared and attached to the primers by the procedure described in WO-A-80/02931.

The predetermined nucleic acid was then dehybridized from the purification reagent by heating to 95°C for 5 minutes, followed by rapid centrifugation and collection of the supernatant.

### Amplification of Target Nucleic Acid:

The supernatant from above was mixed with DNA polymerase isolated from a commercially available Thermus aquaticus (about 1 »l containing 4 I.U./»l), and amplification was carried out for 30 consecutive cycles as follows:

| | |
|---|---|
| 70°C rising to 94°C | 1 minute |
| 94°C | 0.5 minute (denature) |
| 94°C lowering to 50°C | 1.25 minutes |
| 50°C | 0.5 minute (hybridize) |
| 50°C rising to 70°C | 0.75 minute |
| 70°C | 1 minute (extend) |

After amplification, the dehybridized products were evaluated by gel electrophoresis using 4% agarose followed by ethidium bromide staining. The results indicated that the predetermined nucleic acid was specifically purified and removed from the crude lysate using the purification reagent, and amplification successfully carried out.

### Example 2: Method to Detect Human β-Globin

This example is similar to Example 1 but it illustrates the practice of the present invention to purify and detect Human β-globin DNA.

Crude whole blood lysate was obtained as described in Example 1.

A purification reagent was prepared using polymeric particles having surface aldehyde groups. These particles were prepared by the following steps: a mixture of 250 ml of 0.5 molar sodium methoxide in methanol, 2-nitropropane (12.5 ml) and an aqueous suspension (3.5 % solids) of poly(styrene-co-vinylbenzyl chloride) (70:30 molar ratio) was refluxed for two hours, then filtered. The particles on the filter were washed with methanol, dried, reswollen in a minimal volume of pyridine and resuspended in water.

A nucleic acid fragment complementary to the target Human β-globin DNA was used having the following sequence:
5'-X-CTCCTGAGGAGAAGTCTGC-3'
wherein X is a free amino group attached through an ethylene glycol spacer arm prepared as described above.

The aldehyde groups on the particles and the aminooligonucleotide were coupled using the procedure described in US-A-4,587,046.

The purification method and the amplification method of Example 1 were followed herein except that in addition to the use of two Human β-globin DNA primers, the two HLA DNA primers of Example 1 (0.2 »molar) were added as Controls.

The Human beta-globin DNA primers had the following sequences:
(+) 5'-X-ACACAACTGTGTTCACTAGC-3'
(-) 5'-X-CAACTTCATCCACGTTGACC-3'
wherein X represents a biotintetraethylene glycol spacer arm prepared and attached as described in WO-A-89/02931 (noted above).

The results indicated that the Human β-globin DNA target was successfully removed from the crude lysate, amplified and detected using ethidium bromide. The HLA DNA was not amplified or detected in this example.

## Claims

1. A method for amplifying at least one single-stranded, targeted nucleic acid in a biological specimen suspected of containing a mixture of nucleic acids using a polymerase chain reaction comprising:
A. contacting the specimen with a purification reagent comprising a nonporous, nonmagnetic particle having directly attached thereto a nucleic acid fragment complementary to a nucleic acid sequence of the targeted nucleic acid, under conditions suitable to form an insoluble hybrid,
B. separating the hybrid from the remainder of the specimen, and
C. amplifying the nucleic acid sequence of interest using a polymerase chain reaction with or without denaturation from the insoluble hybrid.

2. A method for the detection of at least one targeted nucleic acid having two complementary strands present in a biological specimen suspected of containing a mixture of nucleic acids, the method comprising:
A. denaturing the complementary strands in the specimen,
B. contacting the specimen with a purification reagent comprising a nonporous, nonmagnetic particle having directly attached thereto a nucleic acid fragment complementary to a nucleic acid sequence of the targeted nucleic acid, under conditions suitable to form an insoluble hybrid,
C. separating the hybrid from the remainder of the specimen to provide purified targeted nucleic acid,
D. denaturing the hybrid and separating the insoluble complementary fragment from the targeted nucleic acid,
E. contacting a sample of the denatured targeted nucleic acid with first and second primers, one of which is complementary to the nucleic acid sequence of interest and the other which is complementary to the other strand of the targeted nucleic acid so as to form hybridized products of the first and second primers and the complementary nucleic acid strands,
F. forming first and second extension products of the primers in the hybridized product, which extension products, when separated from their complements, can serve as templates for synthesis of the extension products of the primers,
G. separating the primer extension products from the templates on which they were synthesized,
H. contacting the separated extension products and the targeted nucleic acid with additional first and second primers, resulting in amplification of the specific nucleic acid sequence to form complementary products,
I. separating the primer extension products from the complementary products formed in step H,
J. contacting at least one primer extension product separated in step H with an oligonucleotide probe which is labeled for detection and is complementary thereto to form a complementary product of the probe and the primer extension product, and
K. detecting the complementary product formed in step J as an indication of the presence of the targeted nucleic acid in the specimen.

3. The method as claimed in claim 1 or claim 2 wherein the specimen is whole blood or semen.

4. The method as claimed in any of claims 1 to 3 wherein the targeted nucleic acid is HIV-I DNA.

5. The method as claimed in any of claims 1 to 3 wherein the targeted nucleic acid is Human Leukocyte Antigen DNA.

6. The method as claimed in any of claims 1 to 3 wherein the targeted nucleic acid is Human β-globin DNA.

## Patentansprüche

1. Verfahren zur Amplifizierung mindestens einer einzelsträngigen Ziel-Nukleinsäure in einer biologischen Probe, von der angenommen wird, daß sie eine Mischung von Nukleinsäuren enthält, unter Anwendung einer Polymerasenkettenreaktion, bei dem man:
A. die Probe mit einem Reinigungsreagenz in Kontakt bringt, das umfaßt ein nicht poröses, nicht magnetisches Teilchen, an das direkt gebunden ist ein Nukleinsäurefragment, das komplementär ist einer Nukleinsäuresequenz der Ziel-Nukleinsäure, unter Bedingungen, die zur Bildung eines unlöslichen Hybrides geeignet sind, bei dem man
B. das Hybrid von dem Rest der Probe abtrennt, und bei dem man
C. die Nukleinsäuresequenz von Interesse amplifiziert, unter Anwendung einer Polymerasenkettenreaktion mit oder ohne Denaturierung von dem unlöslichen Hybrid.

2. Verfahren zur Bestimmung mindestens einer Ziel-Nukleinsäure mit zwei komplementären Strängen, die in einer biologischen Probe vorhanden sind, von der angenommen wird, daß sie eine Mischung von Nukleinsäuren enthalten, bei dem man:
A. die komplementären Stränge in der Probe denaturiert,
B. die Probe mit einem Reinigungsreagenz in Kontakt bringt, das umfaßt ein nicht poröses, nicht magnetisches Teilchen mit einem direkt hieran gebundenen Nukleinsäurefragment, das komplementär ist einer Nukleinsäuresequenz der Ziel-Nukleinsäure, unter Bedingungen, die zur Bildung eines unlöslichen Hybrides geeignet sind,
C. das Hybrid von dem Rest der Probe trennt, unter Gewinnung gereinigter Ziel-Nukleinsäure, bei dem man
D. das Hybrid denaturiert und das unlösliche komplementäre Fragment von der Ziel-Nukleinsäure abtrennt, bei dem man
E. eine Probe der denaturierten Ziel-Nukleinsäure mit ersten und zweiten Primern in Kontakt bringt, von denen einer komplementär ist der Nukleinsäuresequenz von Interesse und der andere, der komplementär ist dem anderen Strang der Ziel-Nukleinsäure, unter Bildung von hybridisierten Produkten von dem ersten und dem zweiten Primer und den komplementären Nukleinsäuresträngen, bei dem man
F. erste und zweite Extensionsprodukte der Primer in dem hybridisierten Produkt bildet, wobei die Extensionsprodukte, wenn sie von ihren Komplementen getrennt werden, als Template für die Synthese von den Extensionsprodukten der Primer dienen können, bei dem man
G. die Primerextensionsprodukte von den Templaten, auf denen sie synthetisiert wurden, trennt, bei dem man
H. die abgetrennten Extensionsprodukte und die Ziel-Nukleinsäure mit zusätzlichen ersten und zweiten Primern in Kontakt bringt, unter Amplifizierung der spezifischen Nukleinsäuresequenz, unter Bildung komplementärer Produkte, bei dem man
I. die Primerextensionsprodukte von den in der Stufe H gebildeten komplementären Produkten trennt, bei dem man
J. mindestens ein Primerextensionsprodukt, das in der Stufe H abgetrennt worden ist, mit einer Oligonukleotidsonde kontaktiert, die für die Bestimmung markiert ist und dazu komplementär ist, unter Bildung eines komplementären Produktes der Sonde und des Primerextensionsproduktes, und bei dem man
K. das in Stufe J gebildete komplementäre Produkt als ein Anzeichen für das Vorliegen der Ziel-Nukleinsäure in der Probe bestimmt.

3. Verfahren nach Anspruch 1 oder Anspruch 2, bei dem die Probe ganzes Blut oder Samen oder Sperma ist.

4. Verfahren nach einem der Ansprüche 1 bis 3, bei dem die Ziel-Nukleinsäure HIV-I-DNA ist.

5. Verfahren nach einem der Ansprüche 1 bis 3, bei dem die Ziel-Nukleinsäure menschliches Leukozyten-Antigen-DNA ist.

6. Verfahren nach einem der Ansprüche 1 bis 3, bei dem die Ziel-Nukleinsäure menschliches β-Globin-DNA ist.

## Revendications

1. Procédé d'amplification d'au moins un acide nucléique monocaténaire cible dans un spécimen biologique soupçonné de contenir un mélange d'acides nucléiques en utilisant une réaction en chaîne de polymérase, comprenant :
A. la mise en contact du spécimen avec un réactif de purification comprenant une particule non poreuse et non magnétique ayant, directement fixé sur elle, un fragment d'acide nucléique complémentaire d'une séquence d'acide nucléique de l'acide nucléique cible, dans des conditions convenant à la formation d'un hybride insoluble,
B. la séparation de l'hybride du reste du spécimen, et
C. l'amplification de la séquence d'acide nucléique intéressante en employant une réaction en chaîne de polymérase avec ou sans dénaturation de l'hybride insoluble.

2. Procédé pour la détection d'au moins un acide nucléique cible ayant deux brins complémentaires présents dans un spécimen biologique soupçonné de contenir un mélange d'acides nucléiques, le procédé comprenant :
A. la dénaturation des brins complémentaires dans le spécimen,
B. la mise en contact du spécimen avec un réactif de purification comprenant une particule non poreuse et non magnétique ayant, directement fixé sur elle, un fragment d'acide nucléique complémentaire d'une séquence d'acide nucléique de l'acide nucléique cible, dans des conditions convenant à la formation d'un hybride insoluble,
C. la séparation de l'hybride du reste du spécimen afin de fournir un acide nucléique cible purifié,
D. la dénaturation de l'hybride et la séparation du fragment complémentaire insoluble de l'acide nucléique cible,
E. la mise en contact de l'acide nucléique cible dénaturé avec une première et une seconde amorce, dont l'une est complémentaire de la séquence d'acide nucléique intéressante et l'autre est complémentaire de l'autre brin de la séquence d'acide nucléique cible de façon à former des produits hybridés des première et seconde amorces et des brins d'acide nucléique complémentaires,
F. la formation de premier et second produits d'extension des amorces dans le produit hybridé, produits d'extension qui, une fois séparés de leur complément, peuvent servir de matrices pour la synthèse de produits d'extension des amorces,
G. la séparation des produits d'extension d'amorce des matrices sur lesquelles ils ont été synthétisés,
H. la mise en contact des produits d'extension séparés et de l'acide nucléique cible avec les première et seconde amorces supplémentaires, résultant en une amplification de la séquence d'acide nucléique pour former des produits complémentaires,
I. la séparation des produits d'extension d'amorce des produits complémentaires formés dans l'étape H,
J. la mise en contact d'au moins un produit d'extension d'amorce séparé dans l'étape I avec une sonde oligonucléotidique qui est marquée pour la détection et est complémentaire de celui-ci, afin de former un produit complémentaire de la sonde et du produit d'extension de l'amorce, et
K. la détection du produit complémentaire formé dans l'étape J en tant qu'indication de la présence de l'acide nucléique cible dans le spécimen.

3. Procédé selon la revendication 1 ou la revendication 2, dans lequel le spécimen est du sang complet ou du sperme.

4. Procédé selon l'une quelconque des revendications 1 à 3, dans lequel l'acide nucléique cible est de l'ADN de HIV-1.

5. Procédé selon l'une quelconque des revendications 1 à 3, dans lequel l'acide nucléique cible est de l'ADN d'antigène de leucocyte humain.

6. Procédé selon l'une quelconque des revendications 1 à 3, dans lequel l'acide nucléique cible est de l'ADN de β-globine humaine.
